# EUROPEAN PATENT SPECIFICATION

(11) **EP 4 464 174 B1**
(45) Date of publication and mention of the grant of the patent: **09.09.2026**
(21) Application number: 23382457.2
(22) Date of filing: 16.05.2023
(51) Int. Cl.: A23L 33/125, A23L 29/30, A23G 3/34, A23G 3/42

(54) **CARBOHYDRATE-RICH FOOD PRODUCT**
KOHLEHYDRATE-REICH LEBENSMITTEL
PRODUIT ALIMENTAIRE RICHE EN HYDRATES DE CARBONE

(43) Date of publication of application: 20.11.2024
(73) Proprietor: We Are Unusual S.L., 03203 Elche (Alicante) (ES)
(72) Inventor: BELTRÁ LÓPEZ, Alfonso, 03660 NOVELDA (Alicante) (ES)
(74) Representative: Herrero & Asociados, S.L.

(56) References cited:
- CN-A- 107 149 063
- CN-A- 107 149 073
- US-A1- 2021 015 125
- US-A1- 2022 240 558
- STEVENSON EMMA J ET AL: "A comparison of isomaltulose versus maltodextrin ingestion during soccer-specific exercise", EUROPEAN JOURNAL OF APPLIED PHYSIOLOGY, SPRINGER VERLAG, HEIDELBERG, DE, vol. 117, no. 11, 19 September 2017 (2017-09-19), pages 2321 - 2333, XP036372351, ISSN: 1439-6319, [retrieved on 20170919], DOI: 10.1007/S00421-017-3719-5

## Description

### FIELD OF THE INVENTION

The present invention refers generally to the field of nutrition, in particular performance nutrition. More specifically, it refers to a food product, its use as sports supplement and its use for increasing performance during exercise and reducing fatigue during and after exercise.

### BACKGROUND OF THE INVENTION

Athletes participating in endurance sports such as cycling, running, climbing and the like, require energy supplements that are light and easy to carry, quickly consumed and efficiently absorbed by the body and transformed into available energy.

It is well established that carbohydrate ingestion during exercise improves endurance performance during prolonged (>2 h, preferably ≥3 h) exercise, but also during shorter duration exercise. In fact, it has been shown in several studies that the intake of carbohydrates (also referred to as CHO hereinafter) during exercise results in maintaining sports performance for longer, delaying the time to exhaustion.

A direct correlation has been revealed between the amount of carbohydrates ingested and performance and recovery: the more carbohydrates ingested, the higher the performance and the better the post-exertion recovery. However, the turning point is the amount of carbohydrates that the body can assimilate without causing adverse gastrointestinal (Gl) disturbances and without affecting performance.

A variety of adverse GI symptoms/problems may occur during exercise, which may be attributed to disorders of the upper (esophagus and stomach) or lower (small bowel and colon) GI tract. Upper GI symptoms include, for example, reflux, nausea, bloating, and upper abdominal cramping. Lower GI complaints comprise, amongst others, lower abdominal cramping, the urge to defecate, increased frequency of bowel movements, flatulence, and diarrhoea. Many of these symptoms have been shown to be exacerbated with fluid intake, and specifically carbohydrate intake.

In line with these negative GI symptoms related to carbohydrate intake and given the fact that previous research has shown that a single source of carbohydrate can only be oxidized at a maximum of 1 g/min or 60g/h, the American College of Sports Medicine (ACSM) current recommendations for carbohydrate intake during exercise is 30-60 g CHO/h (American College of Sports Medicine, American Dietetic Association, and Dietitians of Canada. Med Sci Sports Exerc 32: 2130-2145, 2000), and this amount can be exceeded when several carbohydrate sources (e.g., fructose and glucose) are used.

A variety of energy supplements, typically containing one or more carbohydrate such as glucose, fructose, high fructose corn syrup, sucrose, maltodextrin and the like, exist on the market, including sport drinks, energy bars and concentrated viscous liquids, such as gels. Different sport compositions have been described in, for example, US 2021/015125 A1, WO 2007/083117 A1 and US 2006/0193949 A1.

However, even though there is a great variety of supplements comprising one or more carbohydrates, negative GI issues are still reported by the athletes, especially when they ingest large amounts of carbohydrates (e.g., 90 g or more per hour).

Therefore, there is still a need in the state of the art of providing food products for increasing performance and sports recovery in a healthy way and reducing GI adverse symptoms. Surprisingly, the authors of the present invention have developed said food products.

### OBJECT OF THE INVENTION

A **first aspect** of the present invention refers to a food product comprising:
a) isomaltulose;
b) fructose; and
c) and at least one other carbohydrate selected from the group consisting of maltodextrin, amylopectin, cyclodextrin, dextrose, and glucose;

wherein the content of isomaltulose and fructose together is 35%-70% by weight of the total weight of carbohydrates (w/w_{tCHO}), being at least 10% w/w_{tCHO} of isomaltulose and at least 25% w/wt_{CHO} of fructose;
wherein the content of the at least one other carbohydrate selected from the group consisting of maltodextrin, amylopectin, cyclodextrin, dextrose and glucose is 30%-65% w/w_{tCHO}; and
wherein the food product comprises at least 40% of carbohydrates by weight of the total weight of the food product (w/wₜ). According to the present invention, said food product is in the form of a gel, energy bar, beverage, powder, or gummy candy.

A **second aspect** of the present invention refers to a method for preparing the food product of the first aspect of the invention comprising mixing the ingredients of the food product and, optionally, formulating it into the desired form (e.g., gel).

A **third aspect** of the present invention refers to a use of the food product of the first aspect of the invention as sports supplement.

A **fourth aspect** of the present invention refers to a use of the food product of the first aspect of the invention for increasing performance during exercise.

A **fifth aspect** of the present invention refers to a use of the food product of the first aspect of the invention for reducing fatigue during and/or after exercise.

Other objects, features, advantages and aspects of the present application will become apparent to those skilled in the art from the following description and appended claims.

### DETAILED DESCRIPTION OF THE INVENTION

As used herein, the singular forms "a," "an" and "the" include their corresponding plural forms unless the context clearly indicates otherwise. Unless defined otherwise, all the technical and scientific terms used herein have the same meaning as commonly understood by a person skilled in the art to which this invention belongs.

In a **first aspect,** the present invention refers to refers to a food product comprising:
a) isomaltulose;
b) fructose; and
c) and at least one other carbohydrate selected from the group consisting of maltodextrin, amylopectin, cyclodextrin, dextrose, and glucose;

wherein the content of isomaltulose and fructose together is 35%-70% by weight of the total weight of carbohydrates (w/w_{tCHO}), being at least 10% w/wt_{CHO} of isomaltulose and at least 25% w/wt_{CHO} of fructose;
wherein the content of the at least one other carbohydrate selected from the group consisting of maltodextrin, amylopectin, cyclodextrin, dextrose and glucose is 30%-65% w/w_{tCHO}; and
wherein the food product comprises at least 40% of carbohydrates by weight of the total weight of the food product (w/wₜ). According to the present invention, said food product is in the form of a gel, energy bar, beverage, powder, or gummy candy.

Thus, the food product of the present invention comprises at least three different carbohydrates, in particular, (a) isomaltulose, (b) fructose and (c) a carbohydrate selected from maltodextrin, amylopectin, cyclodextrin, dextrose and/or glucose. Many sports supplements comprise fructose and other carbohydrate of (c) (e.g., glucose), but none, to the knowledge of the inventors, comprises also isomaltulose, and even less in the proportions herein described.

The term "food product" as used herein refers particularly to any edible composition suitable for human or animal consumption. Such a product can be in solid, liquid or semisolid (e.g., gel) form.

The food product of the present invention can be formulated in many different forms so it is very versatile and can be adapted to the necessities of the consumer. Thus, according to the present invention, the food product is in the form of gel, energy bar, powder, beverage or drink (e.g., sport drink), or candy (e.g., hard candy or gummy candy). Each formulation has it owns advantages. For example, gels are generally very well-liked by athletes, since they are easy to carry, easy to consume and - due to their moistness - easy to swallow, even during exercises. Thus, in a preferred embodiment, the food product of the invention is in the form of a gel, i.e., it is a gel food product.

For the purpose of the present invention, a "gel" is a product that is a semi-solid state in the approximately range between 10 and 40 °C, more particularly, it has a G' (storage modulus which concerns the solid part of the material) higher than G" (loss modulus which concerns the liquid-like response on the material).

The term "beverage" intends to include liquids and syrups, as well as powder formulations to be dissolved in water or another liquid component (for example orange juice, tea, coffee, fruit and/or vegetable smoothie, another dietary supplement based on mineral salts in liquid form, etc.) for the preparation of instant beverages. When the food product is in powder form, it can be easily prepared before use by dissolving and/or mixing the powder in the appropriate liquid chosen by the user. If it is in the form of a drink, it is ready for use and therefore immediately usable by the user.

In a particular embodiment according to any of the embodiments of the first aspect of the invention, the content of isomaltulose and fructose together is 35%-70% w/w_{tCHO}, being 10%-45% w/w_{tCHO}, preferably 20%-35% w/w_{tCHO}, of isomaltulose and 25%-60% w/w_{tCHO}, preferably 30%-50% w/wt_{CHO} of fructose.

As shown in the Examples, advantageously, the addition of isomaltulose and, particularly, the concentrations and proportions of the different components of the food product of the present invention, reduces the GI disturbances and the perception of fatigue, and increases the sensation of energy in comparison with, for example, food products comprising other carbohydrates, e.g., only fructose and maltodextrin (see Example 2) and only fructose, glucose and dextrose (see Example 3).

As shown in the Examples, said advantages are exacerbated when the food product comprises (within component c)) (i) dextrose and/or glucose; and (ii) maltodextrin, amylopectin, cyclodextrin or a combination thereof, and especially when the content of dextrose and/or glucose is 15-30% w/w_{tCHO}, and the content of maltodextrin, amylopectin, cyclodextrin or a combination thereof is 15-35% w/w_{tCHO}.

Thus, in a particular embodiment according to any one of the previous embodiments, the food product of the invention comprises:
i. dextrose and/or glucose; and
ii. maltodextrin, amylopectin, cyclodextrin or a combination thereof.

More preferably, the content of dextrose and/or glucose is 15-30% w/w_{tCHO}, and the content of maltodextrin, amylopectin, cyclodextrin or a combination thereof is 15-35% w/w_{tCHO}.

Furthermore, as shown in the Examples, food products according to the invention wherein the sum of the content of a) and b) is equal or higher to c) result in a higher increase in the sensation of energy, a lower perception of fatigue and less GI disturbance. Thus, in a particular embodiment, the sum of the content of a) and b) is equal or higher to c), preferably the sum of the content of a) and b) is higher to c), as occurs, for example, in the food products SM3 and SM4.

In a preferred embodiment, the food product comprises the percentages of a), b) and c) defined in any of the food products used in the Examples, e.g., the percentages of the food products SM1, SM2, SM3 or SM4.

In a particular embodiment according to any one of the previous embodiments, the food product comprises at least 50% of carbohydrates w/wₜ, preferably from 50% to 95% or 100% w/wₜ, more preferably from 60% to 80% w/wₜ. Like this, a high carbohydrate content food product is provided, which can easily be taken by the athlete as a concentrate source of carbohydrates.

In a particular embodiment, when the food product is gel, the content of carbohydrates is 40%-60% w/wₜ, or when the food product is a candy, e.g., gummy candy, the content of carbohydrates is 60%-80% w/wₜ, or when the food product is a powder the content of carbohydrates is 90-95% w/wₜ or 65%-70% w/wₜ.

In a preferred embodiment according to any one of the embodiments of the first aspect of the invention, the content of carbohydrates w/wₜ refers to the carbohydrates a), b) and c) as defined above.

Without wishing to be bound by any particular theory, the inventors believe that the particular qualitative and quantitative composition of the food product of the invention reduces or prevents the saturation or collapse of the intestinal transporter SGLT 1, SGLT 2 and GLUT5 and, therefore, the food products of the present invention improve the ability to absorb high amounts of carbohydrates in a short period of time, resulting in higher energy and less fatigue, and also decrease the risk of GI problems.

In the food product of the invention, glucose and fructose can be provided in liquid form (syrup). Thus, in a particular embodiment according to any one of the previous embodiments, the glucose is glucose and/or glucose syrup, and/or the fructose is fructose and/or fructose syrup.

The food product of the present invention can be supplemented with vitamins, and/or minerals and/or electrolytes to additionally enrich it. Thus, in a particular embodiment according to any one of the previous embodiments, the food product further comprises (a) vitamins and/or (b) minerals and/or electrolytes. Preferably in amounts that correspond to at least 10 % of the recommended daily dose.

In a preferred embodiment of the invention, the vitamin is selected from the group consisting of Vitamin C, Vitamin E, Vitamin B2, Vitamin B6, and combinations thereof.

In another preferred embodiment according to any one of the preceding embodiments. The electrolyte and/or mineral is selected from the group consisting of potassium, sodium, magnesium, iron, zinc, calcium, and combinations thereof, more preferably potassium, sodium and/or magnesium. These compounds may contribute to the taste of the food product, may be helpful to replenish the body with compounds that the person is constantly losing due to the generation of sweat during exercise.

Furthermore, the food product may comprise additional food ingredients. Thus, in a particular embodiment according to any one of the previous embodiments, the food product further comprises one or more other food ingredients selected from natural or artificial flavoring agents, natural or artificial coloring agents, non-nutritive sweeteners, preservatives, acidity regulators, proteins, amino acids, fats, gelling agents, antioxidants, caffeine, ginseng, guarana, fruit juice, and taurine. These agents may improve the food product of the present invention with respect to many properties, such as taste, consistency, colour, and stability during storage, digestibility, and many more that are known to those of skill in the art.

The antioxidants can be provided as a fruit extract, particularly a fruit extract rich in polyphenols (e.g., sour cherry extract).

In a particular embodiment, the food product further comprises proteins and/or caffeine and/or antioxidants and/or gelling agents. The protein may include animal and/or vegetable protein, and hydrolysates thereof. For example, soy protein, pea protein, chicken egg protein, meat protein, milk-derived protein (e.g., whey protein). Gelling agents are required to formulate the food product as a gel or gummy candy.

In a particular embodiment, when the food product is a powder the content of carbohydrates is 65%-70% w/wₜ, and it further comprises 20%-25% w/wₜ of protein and 5%-10% w/wₜ antioxidants. This powder form is especially suitable for recovery after exercise. Preferably, this powder consists of 65%-70% w/wₜ, 20%-25% w/wₜ of protein and 5%-10% w/wₜ antioxidants, summing up all its ingredients 100% w/wₜ.

The energy density of the food product is not critical for its effectiveness. However, a high energy density has the advantage that less food product needs to be ingested to replenish carbohydrates as fuel to the body. The food product of the present invention can be formulated to supply a broad range of energy density. Thus, in a particular embodiment according to any one of the previous embodiments, the food product has an energy density of 500-1700 kJ/100 g, preferably 900-1200 kJ/100 g.

To be easily consumable - for example during a competition or in between competitions - the serving size of the food product of the present invention is preferably relatively small. In a preferred embodiment, the food product has a serving size of 10-120 g or ml, preferably 15-110 g or ml, most preferred 50-100 g or ml.

The food products of the present invention can be packaged into flexible, tearable pouches, packets or tubes that are easily torn open such that the contents can be directly administered into the subject, e.g., athletes, mouth with a single squeeze. Thus, in another embodiment, the food product of the invention is packaged, particularly packaged in a package comprising from 10 to 120 g or ml, preferably from 15 to 110 g or ml, more preferably 50 to 100 g or ml, of the food product. Thus, the present invention also refers to a package comprising from 10 to 120 g or ml, preferably from 15 to 110 g or ml, more preferably 50 to 100 g or ml of the food product of the first aspect of the invention. Preferably, the package is a flexible, tearable pouch, packet or tube.

While the subject matter of the present invention is primarily intended for athletes as sports supplement, it is clear, that it can be used by anybody in need of carbohydrate supply. For example, the food product of the present invention can be very well used as transportable food for people, who do not wish to carry too heavy provisions, for example during long term trips. The food product of the present invention may equally well be used by people to provide the body with carbohydrates for example before or during an examination in school or at university.

The food product according to any of the embodiments of the first aspect of the invention can be taken orally before and/or after physical or sports activity.

Preferably, the food product according to any of the embodiments of the first aspect of the invention is a sports supplement or a food supplement.

The food product of the present invention can be prepared by any method known in the art. A **second aspect** of the present invention refers a method for preparing the food product of any one of the embodiments of the first aspect of the invention comprising mixing the ingredients and, optionally, formulating the product. With the formulation step, the product is formulated into the desired form. This can be done by any method known by the skilled in the art.

When the food product is a powder, the ingredients are mixed in powder form. As mentioned above, for consumption, the powder can be dissolved in water or any other liquid.

In a particular embodiment, the method for preparing a gel or a gummy comprises:
a) mixing the ingredients to form a mass,
b) heating the mass to obtain a gel, and
c) filling a mold or container (e.g., pouch) with the gel.

The primary purpose of step a) is to have a smooth and homogenous mass, which is achieved though good stirring, so all ingredients are properly dissolved. The primary purpose of step b), which is the heating process, is to have a microbiological control and to lower the viscosity of the product to facilitate the filling process of step c). The filing process can be a hot-filling of the mass (e.g., gel) into pouches, followed by the hot-sealing of the pouches.

The temperature of the heating step can be easily determined by the skilled in the art and it depends on the ingredients of the food product. For example, if the food product comprises pectin, gelling occurs at a temperature of at least 80°C, preferably 80°C-90°C, more preferably 85°C, thus, heating will be carried out at any of these temperatures.

A **third aspect** of the present invention refers to a use of the food product of any one of the embodiments of the first aspect of the invention as sports supplement or food supplement. A preferred embodiment refers to the use as sports supplement or food supplement to provide an increased performance (in particular, increased endurance performance), energy, recovery, power, and/or strength.

The third aspect of the invention also refers to a use of the food product of any of the embodiment of the first aspect of the invention for the preparation of a sports supplement or a food supplement. A preferred embodiment refers to the use for the preparation of a sports supplement or food supplement to provide an increased performance (in particular increased endurance performance), energy, recovery, power, and/or strength. More preferably, the invention refers to the use of the food product of the present invention for increasing performance, energy, power, and/or strength during exercise; or improving recovery after exercise. Moreover, the invention refers to the food product according to any one of the embodiments of the first aspect of the invention for use in increasing performance, energy, power, and/or strength during exercise; or improving recovery after exercise.

As shown in the Examples, the present invention increases performance, in particular endurance performance. The food product of the invention increases performance even to a higher level than other sport food products do and, surprisingly, while reducing the GI adverse effects usually produced by carbohydrate compositions. Thus, a **fourth aspect** of the present invention refers to a use of the food product of any of the embodiments of the first aspect of the invention for increasing performance (particularly endurance performance), energy, recovery, power, and/or strength.

The fourth aspect of the invention also relates to a method for increasing performance (preferably endurance performance), energy, recovery, power, and/or strength of a subject, the method comprising consuming (e.g., eating or drinking) the food product of any of the embodiment of the first aspect of the invention. Particularly, the subject consumes the food product before, during and/or after the subject engages in exercise.

In a particular embodiment, an effective amount of the food product is consumed by the subject. The effective amount is easily determinable by the skilled in the art.

In a preferred embodiment of the fourth aspect of the invention, the uses and methods are for increasing performance, particularly endurance performance, energy, power, and/or strength, during exercise, and for improving recovery after exercise.

Thus, a **fifth aspect** of the present invention refers to a use of the food product of any of the embodiment of the first aspect of the invention for reducing fatigue during exercise and/or after exercise. Likewise, the invention refers to the food product of any of the embodiment of the first aspect of the invention for use in reducing fatigue during exercise and/or after exercise.

Likewise, it refers to a method for reducing fatigue of a subject during exercise and/or after exercise comprising the consumption of the food product of any of the embodiment of the first aspect of the invention. Particularly, the subject consumes the food product before, during and/or after the subject engages in exercise. In a particular embodiment, an effective amount of the food product is consumed. The effective amount is easily determinable by the skilled in the art.

In a particular embodiment of the third, fourth and fifth aspects of the invention, the food product of the present invention is used/consumed in an amount that corresponds to an ingestion of at least 30 g carbohydrates per hour, preferably at least 60 g carbohydrates per hour, more preferably at least 90 g carbohydrates per hour.

In another particular embodiment of the third, fourth and fifth aspects of the invention, the food product of the present invention is used/consumed in an amount that corresponds to an ingestion of fructose molecules from 30% to 60%, preferably from 33% to less than 55%, of the total of carbohydrates (w/w_{tCHO}). More particularly, the food product of the present invention is used/consumed in an amount that corresponds to a ratio, preferably weight ratio, glucose molecules:fructose molecules of 1:0.5 to 1:1, preferably 1:05 or 1:0.8, and more preferably 1:1. These amounts maximize the intestinal absorption of these carbohydrates by their specific intestinal receptors (e.g., SGLT 1, SGLT 2 for glucose, and GLUT5 for fructose).

In another particular embodiment of the third, fourth and fifth aspects of the invention, when the sport is cycling, the food product of the present invention may also be used to improve cycling cadence, for example measured in revolutions per minute and/or to decrease ratings of perceived exertion (RPE).

Interestingly, as shown in the Examples, with the food product of the present invention (endurance) performance is increased and fatigue is reduced, even more than when using known food products. Surprisingly, the particular combination of carbohydrates and their percentages of the present invention allows that the high carbohydrate content of the food product of the present invention is well tolerated by the body, so that problems of the GI tract that one would normally expect after a high level of carbohydrate intake during exercise are at least partially avoided. Moreover, the GI tolerance for carbohydrates is increased. Thus, the third, fourth and fifth aspect of the invention also refer to the uses and methods therein described (1) while reducing the GI adverse symptoms exacerbated by carbohydrate intake, and/or (2) while improving GI comfort, and/or (3) without any increased of GI problems/symptoms.

The problems with the GI tract as mentioned above are not particularly limited but are preferably selected from the group consisting of upper abdominal problems such as reflux, heartburn, bloating, upper abdominal cramps, vomiting, nausea; lower abdominal problems such as intestinal cramps, flatulence, urge to defecate, left abdominal pain, right abdominal pain, loose stool, diarrhoea; or systemic problems such as dizziness, headache, muscle cramp or urge to urinate.

For exercise in general, but in particular for competitive exercise it is essential that the body has blood sugar available for the muscles to burn at all times. In particular at the end of the competition absence of available blood sugar must be avoided, or the athlete will run out of energy. The subject matter of the present invention is well suited to prevent this. According to one embodiment of the present invention the food product of the present invention can be used to allow for an enhanced blood sugar maintenance late in exercise.

Finally, the food product of the present invention may be used to provide faster energy delivery, in particular to working muscles, and/or to provide more sustained energy to muscles. Both effects will contribute to an optimal performance of an athlete.

### EXAMPLES

Specific embodiments of the invention that serve to illustrate the invention without limiting the scope thereof are described in detail below.

### EXAMPLE 1.- Comparative assay

A comparative assay was carried out between three different food products according to the present invention: SM1, SM2 and SM3. All products were in the form of a gel, which was ingested directly during sports practice.

Method:
- Ingest the gels that are appropriate to achieve a nutritional strategy of 110 g of carbohydrates every hour of effort.
- Evaluate the following items by means of a questionnaire: Gastrointestinal Comfort, Sensation Energy, Perception of fatigue at the end of an effort for more than 3 hours. A scale of 1 to 7 was use, being 1 the minimum GI comfort/energy/fatigue, and 7 the maximum GI comfort/energy/fatigue.
- The training sessions lasted more than 3 hours.
- Sample: 30 professional cyclists

The compositions for 100 g of carbohydrates of SM1, SM2 and SM3 are given in Table 1, as w/w_{tCHO}.

**Table 1**

| SM1 | SM2 | SM3 |
|---|---|---|
| 25% Isomaltulose | 25% Isomaltulose | 25% Isomaltulose |
| 25% Fructose | 25% Fructose | 33% Fructose |
| 50% Maltodextrin | 25% Maltodextrin | 21% Maltodextrin |
| | 25% Dextrose | 21% Dextrose |

The results are shown in Table 2.

| Table 2 | SM1 | SM2 | SM3 |
|---|---|---|---|
| Gastrointestinal Comfort | 4.5 | 5 | 6.5 |
| Sensation Energy | 5 | 5 | 6 |
| Perception of fatigue | 5.5 | 5 | 4.5 |

It can be concluded that:
- SM3 gel is better assimilated by the body giving less symptoms of GI discomfort.
- The sensation of energy with SM3 gel is more than with SM1 and SM2
- The sensation of fatigue with SM3 is less than with SM1 and SM2.
- The use of more than one ingredient of glucose and/or glucose polymers (glucose, dextrose, cyclodextrin, syrups, sucrose, maltodextrin, etc.) improves GI comfort.

### EXAMPLE 2.- Comparative assay

A comparative assay was carried out between a food product according to the present invention (SM4) and a commercial food product (Brand X). Both were formulated as powder, and they were reconstituted in water prior to their consumption.

The compositions for 100g of carbohydrates are given in Table 3, as w/w_{tCHO}.

**Table 3**

| SM4 | Brand X |
|---|---|
| 20% Isomaltulose | 60% Maltodextrin |
| 47% Fructose | 40% Fructose |
| 16,5% Dextrose/Glucose | |
| 16,5% Maltodextrin | |

Method:
- Two drinks both with 500ml with 100g of carbohydrates each.
- The evaluated items and their scales were the same as in Example 1, taste was also evaluated with the same scale.
- Training sessions were of more than 3 hours duration.
- The nutritional strategy was of 110g of carbohydrates every hour of effort.

Sample: 30 professional cyclists

The results are shown in Table 4.

| Table 4 | SM4 | Brand X |
|---|---|---|
| Gastrointestinal Comfort | 6.5 | 4 |
| Taste | 5 | 5 |
| Sensation Energy | 6 | 5 |
| Perception of fatigue | 5 | 6 |

From this table, it can be concluded that:
- The SM4 drink is better assimilated by the organism giving less symptoms of GI discomfort.
- The sensation of energy with the SM4 drink is higher and the sensation of fatigue is less than with the commercial drink (Brand X).
- The above-mentioned advantages are achieved without affecting the taste of the food product.

### EXAMPLE 3.- Comparative assay in gel form

A comparative assay was carried out between a food product according to the present invention (SM3) and two commercial food products (Y and Z). All were formulated as gel. The compositions for 100g of carbohydrates are given in Table 5, as w/w_{tCHO}. The assay was performed as in Example 1 but using the gels of Table 5.

**Table 5**

| Gel SM3 (as above) | Gel Y | Gel Z |
|---|---|---|
| 25% Isomaltulose | 60% Glucose/Dextrose | 50% maltodextrin |
| 33% Fructose | 40% Fructose | 25% cyclodextrin |
| 21% Maltodextrin | | 15% corn syrup |
| 21% Dextrose | | 10% sugar |

The results are shown in Table 6.

| Table 6 | Gel SM3 | Gel Y | Gel Z |
|---|---|---|---|
| Gastrointestinal Comfort | 6.5 | 4 | 2 |
| Sensation Energy | 6 | 5 | 4 |
| Perception of fatigue | 4.5 | 6 | 7 |

From this table, it can be concluded that:
- The SM3 gel is better assimilated by the organism giving less symptoms of gastric discomfort.
- The sensation of energy with the SM3 gel is higher and the sensation of fatigue is lower than with the BRAND X and Y gel.
- Fructose must be present in the composition to reduce the GI problems.

## Claims

1. A food product comprising:
a) Isomaltulose;
b) Fructose; and
c) at least one other carbohydrate selected from the group consisting of maltodextrin, amylopectin, cyclodextrin, dextrose, and glucose;
wherein the content of isomaltulose and fructose together is 35%-70% by weight of the total weight of carbohydrates (w/w_{tCHO}), being at least 10% w/w_{tCHO} of isomaltulose and at least 25% w/w_{tCHO} of fructose;
wherein the content of the at least one other carbohydrate selected from the group consisting of maltodextrin, amylopectin, cyclodextrin, dextrose and glucose is 30%-65% w/w_{tCHO};
wherein the food product comprises at least 40% of carbohydrates by weight of the total weight of the food product (w/wₜ), and
wherein the food product is in the form of gel, energy bar, beverage, powder, or gummy candy

2. The food product according to claim 1, wherein the content of isomaltulose and fructose together is 35%-70% w/w_{tCHO}, being 10%-45% w/w_{tCHO} of isomaltulose and 25%-60% w/w_{tCHO} of fructose.

3. The food product according to claim 1 or 2, wherein the food product comprises:
i) dextrose and/or glucose; and
ii) maltodextrin, amylopectin, cyclodextrin or a combination thereof.

4. The food product according to the preceding claim, wherein the content of dextrose and/or glucose is 15-30% w/w_{tCHO}, and the content of maltodextrin, amylopectin, cyclodextrin or a combination thereof is 15-35% w/w_{tCHO}.

5. The food product according to any one of the preceding claims, wherein the composition comprises at least 50% of carbohydrates w/wₜ, preferably from 50% to 95% w/wₜ.

6. The food product according to any one of the preceding claims, wherein the sum of the content of a) and b) is equal or higher than the one of c).

7. The food product according to any one of the preceding claims, wherein the glucose is glucose or glucose syrup and/or the fructose is fructose or fructose syrup.

8. The food product according to any one of the preceding claims, wherein the food product further comprises vitamins, preferably Vitamin C, Vitamin E, Vitamin B2 and/or Vitamin B6; and/or further comprises minerals and/or electrolytes, preferably potassium, sodium and/or magnesium.

9. The food product according to any one of the preceding claims, wherein the food product has an energy density of 500-1700 kJ/100 g.

10. The food product according to any one of the preceding claims, wherein the food product is in the form of gel, energy bar, powder, or gummy candy.

11. Method for preparing the food product of any one of claims 1-10, comprising the following steps:
a) mixing the ingredients,
b) optionally, formulating the food product.

12. Use of the food product according to any one of claims 1-10 as sports supplement or food supplement.

13. Use of the food product according to any one of claims 1-10 for increasing performance, energy, power, and/or strength during exercise; or for reducing fatigue during exercise and/or after exercise; or for improving recovery after exercise.

14. Use according to claim 12 or 13, wherein the food product is used in an amount that corresponds to an ingestion of at least 30 g carbohydrates/h, preferably at least 60 g carbohydrates /h, more preferably at least 90 g carbohydrates/h.

## Patentansprüche

1. Lebensmittelprodukt, umfassend:
a) Isomaltulose;
b) Fructose und
c) mindestens ein anderes Kohlenhydrat ausgewählt aus der Gruppe bestehend aus Maltodextrin, Amylopektin, Cyclodextrin, Dextrose und Glucose;
wobei der Gehalt an Isomaltulose und Fructose zusammen 35 bis 70 Gew.% des Gesamtgewichts der Kohlenhydrate (Gew./Gew._{tCHO}) beträgt, und mindestens 10 % Gew./Gew._{tCHO} Isomaltulose und mindestens 25 % Gew./Gew._{tCHO} Fructose beträgt;
wobei der Gehalt des mindestens einen anderen Kohlenhydrats ausgewählt aus der Gruppe bestehend aus Maltodextrin, Amylopektin, Cyclodextrin, Dextrose und Glucose 30 % bis 65 % Gew./Gew._{tCHO} beträgt;
wobei das Lebensmittelprodukt mindestens 40 Gew.% Kohlenhydrate umfasst, bezogen auf das Gesamtgewicht des Lebensmittelprodukts (Gew./Gew.ₜ), und
wobei das Lebensmittelprodukt in Form von Gel, Energieriegel, Getränk, Pulver oder Gummibonbon vorliegt.

2. Lebensmittelprodukt nach Anspruch 1, wobei der Gehalt an Isomaltulose und Fructose zusammen 35 % bis 70 % Gew./Gew._{tCHO} beträgt und 10 % bis 45 % Gew./Gew._{tCHO} Isomaltulose und 25 % bis 60 % Gew./Gew._{tCHO} Fructose beträgt.

3. Lebensmittelprodukt nach Anspruch 1 oder 2, wobei das Lebensmittelprodukt umfasst:
i) Dextrose und/oder Glucose; und
ii) Maltodextrin, Amylopektin, Cyclodextrin oder eine Kombination davon.

4. Lebensmittelprodukt nach den vorhergehenden Ansprüchen, wobei der Gehalt an Dextrose und/oder Glucose 15 bis 30 % Gew./Gew._{tCHO} beträgt und der Gehalt an Maltodextrin, Amylopektin, Cyclodextrin oder einer Kombination davon 15 bis 35 % Gew./Gew._{tCHO} beträgt.

5. Lebensmittelprodukt nach einem der vorhergehenden Ansprüche, wobei die Zusammensetzung mindestens 50 % Kohlenhydrate Gew./Gew.ₜ, vorzugsweise 50 % bis 95 % Gew./Gew ₜ umfasst.

6. Lebensmittelprodukt nach einem der vorhergehenden Ansprüche, wobei die Summe des Gehalts an a) und b) gleich oder größer als derjenige von c) ist.

7. Lebensmittelprodukt nach einem der vorhergehenden Ansprüche, wobei die Glucose Glucose oder Glucosesirup ist, und/oder die Fructose Fructose oder Fructosesirup ist.

8. Lebensmittelprodukt nach einem der vorhergehenden Ansprüche, wobei das Lebensmittelprodukt des Weiteren Vitamine umfasst, vorzugsweise Vitamin C, Vitamin E, Vitamin B2 und/oder Vitamin B6; und/oder des Weiteren Mineralien und/oder Elektrolyte umfasst, vorzugsweise Kalium, Natrium und/oder Magnesium.

9. Lebensmittelprodukt nach einem der vorhergehenden Ansprüche, wobei das Lebensmittelprodukt eine Energiedichte von 500 bis 1700 kJ/100 g aufweist.

10. Lebensmittelprodukt nach einem der vorhergehenden Ansprüche, wobei das Lebensmittelprodukt in Form von Gel, Energieriegel, Pulver oder Gummibonbon vorliegt.

11. Verfahren zur Herstellung des Lebensmittelprodukts nach einem der Ansprüche 1 bis 10, umfassend die folgenden Schritte:
a) Mischen der Bestandteile,
b) gegebenenfalls Formulieren des Lebensmittelprodukts.

12. Verwendung des Lebensmittelprodukts nach einem der Ansprüche 1 bis 10 als Sportergänzungsmittel oder Nahrungsergänzungsmittel.

13. Verwendung des Lebensmittelprodukts nach einem der Ansprüche 1 bis 10 zur Steigerung von Leistungsfähigkeit, Energie, Kraft und/oder Stärke während körperlicher Betätigung; oder zum Reduzieren von Ermüdung während körperlicher Betätigung und/oder nach körperlicher Betätigung; oder zur Verbesserung der Erholung nach körperlicher Betätigung.

14. Verwendung nach Anspruch 12 oder 13, wobei das Lebensmittelprodukt in einer Menge verwendet wird, die einer Einnahme von mindestens 30 g Kohlenhydraten/h, vorzugsweise mindestens 60 g Kohlenhydraten/h, bevorzugter mindestens 90 g Kohlenhydraten/h entspricht.

## Revendications

1. Produit alimentaire comprenant :
a) de l'isomaltulose ;
b) du fructose ; et
c) au moins un autre carbohydrate sélectionné parmi le groupe constitué de maltodextrine, d'amylopectine, de cyclodextrine, de dextrose et de glucose ;
dans lequel la teneur en isomaltulose et en fructose groupés est comprise entre 35 % et 70 % en poids du poids total des carbohydrates (w/w_{tCHO}), laquelle teneur est déclinée selon au moins 10 % w/w_{tCHO} d'isomaltulose et au moins 25 % w/w_{tCHO} de fructose ;
dans lequel la teneur en l'au moins un autre carbohydrate sélectionné parmi le groupe constitué de maltodextrine, d'amylopectine, de cyclodextrine, de dextrose et de glucose est comprise entre 30 % et 65 % w/w_{tCHO} ;
dans lequel le produit alimentaire comprend au moins 40 % de carbohydrates en poids du poids total du produit alimentaire (w/wₜ) ; et
dans lequel le produit alimentaire est sous la forme de gel, de barre énergétique, de boisson, de poudre ou de bonbon gélifié.

2. Produit alimentaire selon la revendication 1, dans lequel la teneur en isomaltulose et en fructose groupés est comprise entre 35 % et 70 % en poids w/w_{tCHO}, laquelle teneur est déclinée selon entre 10 % et 45 % w/w_{tCHO} d'isomaltulose et entre 25 % et 60 % w/w_{tCHO} de fructose.

3. Produit alimentaire selon la revendication 1 ou 2, dans lequel le produit alimentaire comprend :
i) du dextrose et/ou du glucose ; et
ii) de la maltodextrine, de l'amylopectine, de la cyclodextrine ou une combinaison de celles-ci.

4. Produit alimentaire selon la revendication précédente, dans lequel la teneur en dextrose et/ou en glucose est comprise entre 15 % et 30 % w/w_{tCHO} et la teneur en maltodextrine, en amylopectine, en cyclodextrine ou en une combinaison de celles-ci est comprise entre 15 % et 35 % w/w_{tCHO}.

5. Produit alimentaire selon l'une quelconque des revendications précédentes, dans lequel la composition comprend au moins 50 % w/wₜ de carbohydrates, de préférence de 50 % à 95 % w/wₜ.

6. Produit alimentaire selon l'une quelconque des revendications précédentes, dans lequel la somme de la teneur en a) et de la teneur en b) est égale ou supérieure à la teneur en c).

7. Produit alimentaire selon l'une quelconque des revendications précédentes, dans lequel le glucose est du glucose ou du sirop de glucose et/ou le fructose est du fructose ou du sirop de fructose.

8. Produit alimentaire selon l'une quelconque des revendications précédentes, dans lequel le produit alimentaire comprend en outre des vitamines, de préférence de la vitamine C, de la vitamine E, de la vitamine B2 et/ou de la vitamine B6 ; et/ou comprend en outre des minéraux et/ou des électrolytes, de préférence du potassium, du sodium et/ou du magnésium.

9. Produit alimentaire selon l'une quelconque des revendications précédentes, dans lequel le produit alimentaire présente une densité énergétique de 500 à 1700 kJ/100 g.

10. Produit alimentaire selon l'une quelconque des revendications précédentes, dans lequel le produit alimentaire est sous la forme de gel, de barre énergétique, de poudre ou de bonbon gélifié.

11. Procédé pour préparer le produit alimentaire selon l'une quelconque des revendications 1 à 10, comprenant les étapes suivantes :
a) le mélange des ingrédients ; et
b) en option, la formulation du produit alimentaire.

12. Utilisation du produit alimentaire selon l'une quelconque des revendications 1 à 10 en tant que complément alimentaire pour sportifs ou que complément alimentaire.

13. Utilisation du produit alimentaire selon l'une quelconque des revendications 1 à 10 pour augmenter la performance, l'énergie, la puissance et/ou la force pendant l'effort ; ou pour réduire la fatigue pendant l'effort et/ou après l'effort ; ou pour favoriser la récupération après l'effort.

14. Utilisation selon la revendication 12 ou 13, dans lequel le produit alimentaire est utilisé selon une quantité qui correspond à une ingestion d'au moins 30 g de carbohydrates/h, de préférence d'au moins 60 g de carbohydrates/h, de façon davantage préférable, d'au moins 90 g de carbohydrates/h.
